Europäisches Patentamt

European Patent Office

Office européen des brevets

(11) Numéro de publication: **0 178 023**
A2

## DEMANDE DE BREVET EUROPEEN

(21) Numéro de dépôt: 85201616.1

(22) Date de dépôt: 07.10.85

(51) Int. Cl.⁴: **A 61 M 3/00,** A 61 D 7/00,
B 65 D 83/00

(30) Priorité: 08.10.84 CH 4810/84

(43) Date de publication de la demande: 16.04.86
**Bulletin 86/16**

(84) Etats contractants désignés: **AT BE CH DE FR GB IT LI
LU NL SE**

(71) Demandeur: **ECHANGEX S.A., 26, Montbrillant,
CH-1201 Genève (CH)**

(72) Inventeur: **Baldacci, Aldo-Edouard, 16, Avenue
Adrien-Jeandin, CH-1226 Thonex/Geneve (CH)**

(74) Mandataire: **Mabut, Marie-France et al, c/o Bugnion
S.A. 10, route de Florissant Case Postale 375,
CH-1211 Genève 12 - Champel (CH)**

(54) **Dispositif pour l'administration de substances liquides ou solides.**

(57) Il se compose d'une pièce cylindrique (1) filetée à l'une de ses extrémités, d'un manchon fileté (5), d'un piston (2) et d'un ressort de détente (6). Le réglage de la course du piston (5) au moyen du filetage (2) de la pièce cylindrique permet de programmer la quantité d'un liquide à aspirer pour l'administration à un être humain ou à un animal.

Dispositif pour l'administration de substances liquides
ou solides.

La présente invention concerne un dispositif pour
l'administration de substances liquides ou solides
destiné notamment à l'administration de gouttess médicamenteuses prescrites par un médecin ou un vétérinaire
ainsi que l'administration de substances solides telles
que gélules, cachets, etc. à usage vétérinaire.

Il existe sur le marché différents dispositifs pour
prélever et compter les gouttes d'un médicament ou d'un
liquide en général; certains font partie intégrante de
la bouteille même ou du container, d'autres sont vendus
à part et ne sont le plus souvent que de simples
pipettes en verre et caoutchouc. L'utilisation de ces
différents types de compte-gouttes demande une attention soutenue et une précision de mouvements dont les
patients sont souvent incapables. Il arrive fréquemment
que le liquide à prélever ne sorte pas de la bouteille,
mais qu'en secouant celle-ci il en sorte trop en une
seule fois et que le volume de la quantité nécessaire
ne peut pas être contrôlé.

D'autre part lors de l'administration de gouttes ou
d'un médicament liquide à un animal il faut que le
liquide soit déposé le plus profondément possible dans
la gueule de l'animal pour éviter qu'il le regurgite.
Le même est vrai pour des médicaments solides sous
forme de gélules, cachets, etc.

La présente invention permet de pallier ces
inconvénients en proposant un dispositif extrêmement
simple, permettant d'une part de programmer la quantité

2

0178023

d'un éventuel médicament liquide à administrer et d'autre part d'administrer le médicament tout au fond de la gueule d'un animal.

Le dispositif selon l'invention est caractérisé par la clause caractérisante de la revendication 1.

Les avantages de ce dispositif sont: que l'on peut programmer la quantité de l'éventuel médicament liquide en réglant la course du piston; que l'on peut administrer un médicament tout au fond de la gueule d'un animal en utilisant un corps cylindrique long; la simplicité de la construction de ce dispositif; si l'on utilise le dispositif comme compte-gouttes, c'est-à-dire l'administration d'un liquide on choisit une pièce cylindrique dont l'extrémité est tronconique et munie d'un trou tandis que si il s'agit d'une substance solide on choisit une pièce cylindrique dont l'extrémité a la forme d'une encoche déformable élastiquement.

L'invention sera décrite plus en détail à l'aide du dessin annexé.

La figure 1 est une vue en coupe d'une variante compte-gouttes en position de détente.

La figure 2 est une vue en coupe de la figure précédente en position rétractée.

La figure 3 est une vue en plan de la variante pour l'administration de substances solides.

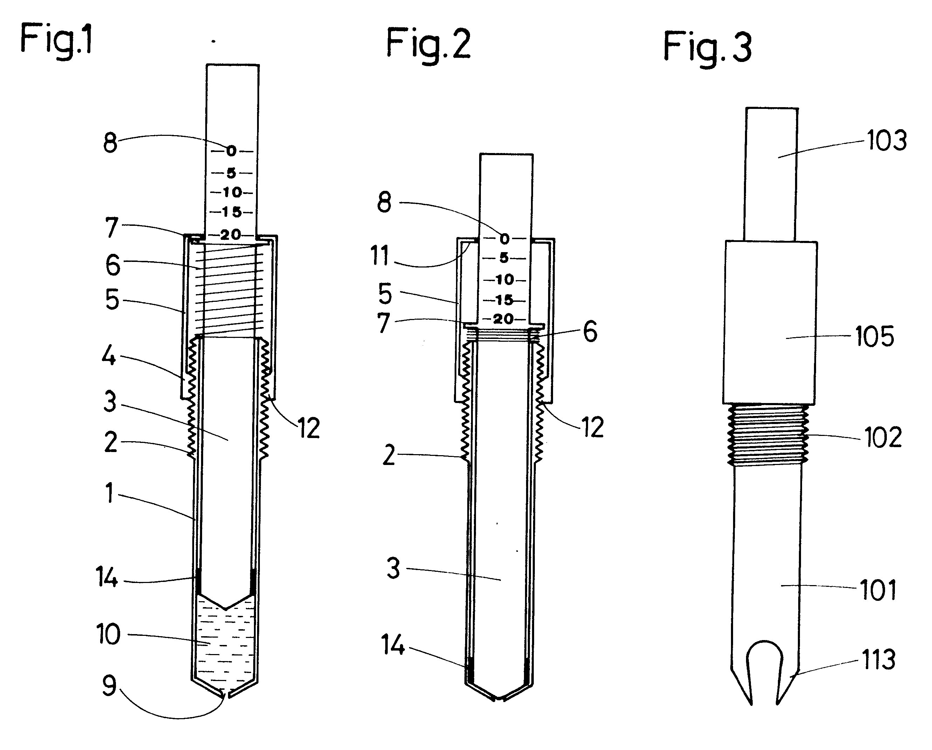

Le dispositif des figures 1 et 2 se compose d'un piston 3 muni d'une embase d'arrêt 7, d'une pièce cylindrique 1 dont l'une de ses extrémités est filetée extérieurement et munie à son autre extémité d'un orifice 9 à son autre extrémité de forme tronconique,d'un manchon 5 présentant un épaulement 11 et dont la paroi intérieure comporte un taraudage 12, d'un ressort de détente 6 et enfin d'une gravure extérieure 8 sur le haut du piston 3 destinée à permettre le préréglage de la quantité de liquide à prélever.

Lorsqu'on visse le manchon 5 en position rétractée (figure 2) sur le cylindre 1 on amène le piston 3 à la hauteur de marquage choisie qui détermine le nombre précis de gouttes ou la quantité du liquide à aspirer dans le cylindre par l'orifice 9. Le ressort 6 a pour fonction de ramener le piston 3 au point de butée déterminé par l' épaulement 8.

Le déplacement étanche du piston 1 dans la pièce cylindrique est assuré par une garniture d'étanchéité 14 ou tout autre moyen connu.

Au lieu de graver la graduation sur le piston on peut la graver sur la pièce cylindrique 1. La longueur soit du filetage 2 de la pièce cylindrique soit du taraudage 12 du manchon 5 permet le réglage de la course du piston 3.

La variante de la figure 3 est destinée à l'administration de gelules, cachets et autres à un animal, la seule différence par rapport au compte-gouttes (figures 1, 2) est que la pièce cylindrique 101 vissée sur le manchon 105 par son filetage 102 est munie à son extrémité libre d'une encoche 113 permettant la préhension d'un cachet ou d'une gelule, laquelle sera

0178023

impulsée à l'intérieur de la gueule d'un animal aussi profondément que possible, à l'aide du piston 103. Bien entendu dans ce cas le réglage de la course du piston n'est pas utile mais pour obtenir cette variante de la figure 3; il suffit de remplacer la pièce cylindrique 1 de la variante de la figure 1 par la pièce cylindrique 101 les autres pièces étant les mêmes.

REVENDICATIONS.

1.    Dispositif   pour   l'administration   de   substances liquides  ou  solides  caractérisé  par  le  fait  qu'il comprend un manchon (5; 105) muni à l'une de ses extrémités d'un épaulement (11), d'une pièce cylindrique (1; 101)  munie à l'une de ses extrémités d'un filetage (2; 102), un piston (3; 103) muni d'une  embase  (7)  monté coulissant de manière étanche dans la pièce cylindrique (1, 101) à travers le manchon (5; 105) et un ressort de détente  (6)  logé  dans le manchon (5; 105), que ledit manchon (5;105) est muni des moyens (12) permettant  le vissage  de  la  pièce  cylindrique  (1;  101)  dans le manchon (5; 105), la profondeur du  vissage  permettant de  programmer  la  longueur de la course du piston (3; 103).

2. Dispositif selon la revendication 1 caractérisé  par le   fait   que   lesdits   moyens   du  manchon  (5,  105) consistent en un taraudage (12) du manchon.

3.  Dispositif  selon  l'une  des revendications 1 ou 2 caractérisé par le fait que  l'extrémité  libre  de  la pièce  cylindrique (101) a la forme d'une encoche (113) déformable élastiquement pour permettre  la  préhension d'une pièce solide.

4.  Dispositif  selon  l'une  des revendications 1 ou 2 caractérisé par le fait que  l'extrémité  libre  de  la pièce cylindrique (1) est tronconique et elle est munie d'un orifice (9) à travers lequel  on  peut  aspirer  a l'aide du piston (3) une quantité de liquide et éjecter cette quantité ou  une  partie  de  cette  quantité  de liquide en actionnant le piston.

2
0178023

5. Dispositif selon la revendication 4 caractérisé par le fait que la pièce cylindrique (1) porte des graduations permettant de programmer la quantité de liquide à aspirer et à éjecter.

6. Dispositif selon la revendication 4 caractérisé par le fait que le piston (3) porte sur sa partie s'étendant au-delà du manchon (5) des graduations (8) permettant de programmer la quantité de liquide à aspirer et à éjecter.

0178023

Fig.3

103
105
102
101
113

Fig.2

8
11
5
7
6
12
2
3
14

0
5
10
15
20

Fig.1

8
7
6
5
4
3
2
1
14
10
9
12

0
5
10
15
20